(19) Europäisches Patentamt
European Patent Office
Office européen des brevets

(11) Veröffentlichungsnummer : **0 278 337 B1**

# (12) EUROPÄISCHE PATENTSCHRIFT

(45) Veröffentlichungstag der Patentschrift : **20.03.91 Patentblatt 91/12**

(51) Int. Cl.[5] : **A01N 37/34,** C07C 255/61

(21) Anmeldenummer : **88101291.8**

(22) Anmeldetag : **29.01.88**

(54) **Mittel gegen pflanzenschädigende Milben auf Basis von Azomethinen des 2,3-Diaminomaleinsäurenitrils.**

(30) Priorität : **11.02.87 DE 3704154**
**06.08.87 DE 3726044**

(43) Veröffentlichungstag der Anmeldung : **17.08.88 Patentblatt 88/33**

(45) Bekanntmachung des Hinweises auf die Patenterteilung : **20.03.91 Patentblatt 91/12**

(84) Benannte Vertragsstaaten : **BE CH DE ES FR GB IT LI NL**

(56) Entgegenhaltungen : **DE-A- 2 438 400**

(56) Entgegenhaltungen : **THE JOURNAL OF ORGANIC CHEMISTRY; Band 39, Nr. 16, Mai-August 1974 R.W.BEGLAND et al.: "Hydrogen Cyanide Chemistry of Diaminomaleonitrile. Heterocyclic Synthesis." Seiten 2341-2350**
**PATENT ABSTRACTS OF JAPAN; unexamined applications, Sektion C, Band 1, Nr. 29, 28 März 1977 THE PATENT OFFICE JAPANESE GOVERNMENT Seite 1678C 76**

(73) Patentinhaber : **BAYER AG**
**W-5090 Leverkusen 1 Bayerwerk (DE)**

(72) Erfinder : **Heidenreich, Holger, Dr.**
**Blankenese 18**
**W-2224 Kuden (DE)**
Erfinder : **Becker, Benedikt, Dr.**
**Metzkausener Strasse 14**
**W-4020 Mettmann (DE)**

**EP 0 278 337 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die Verwendung von teilweise bekannten Azomethinen (= Schiff sche Basen) des 2,3-Diaminomaleinsäurenitrils, als Mittel gegen pflanzenschädigende Milben.

Es ist bereits bekannt, daß Azomethine von 2,3-Diaminomaleinsäurenitril bakterizide, virizide und keimtötende Wirkungen besitzen, wie beispielsweise das Amino-Toluylidenamino-maleinsäurenitril (siehe dazu z.B. JP 5 1151 325).

Weiterhin sind aus der Publikation von R.W. Bagland et al, J. Org. Chem. 39, No. 16 (1974), S. 2341 ff. Azomethine des 2,3-Diaminomaleinsäurenitrils bekannt, welche bestimmte chlorsubstituierte Phenylgruppen enthalten (Amino-2-chlortoluylidenamino-maleinsäurenitril und Amino-2,6-dichlortoluylidenaminomaleinsäurenitril).

Über eine Wirksamkeit der vorgenannten Verbindungsklasse gegen pflanzenschädigende Milben ist bisher jedoch noch nichts bekannt geworden.

Es wurde nun gefunden, daß die teilweise bekannten Azomethine von 2,3-Diamino-maleinsäurenitril der Formel

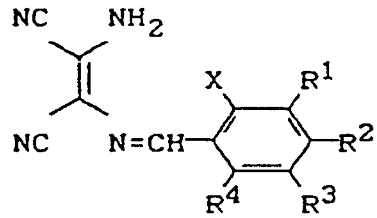

(I)

in welcher

X und $R^4$ gleich oder verschieden sein können und für Fluor, Chlor, Brom, Iod, $CF_3$ oder CN stehen,

$R^1$, $R^2$ und $R^3$ gleich oder verschieden sein können und für Wasserstoff, Alkyl($C_1$-$C_4$), Halogenalkyl-($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)amino, Alkyl($C_1$-$C_4$)sulfonyl, OH, SH, $NH_2$ stehen, sehr stark ausgeprägte Wirkung gegen pflanzenschädigende Milben besitzen.

Die teilweise bekannten Azomethine von 2,3-Diaminomaleinsäurenitril der Formel (I)

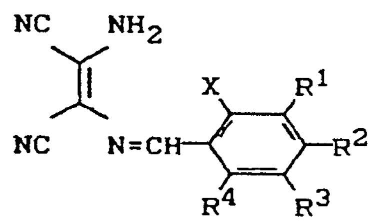

(I)

in welcher

X und $R^4$ gleich oder verschieden sein können und für Fluor, Chlor, Brom, Iod, $CF_3$ oder CN stehen

$R^1$, $R^2$ und $R^3$ gleich oder verschieden sein können und für Wasserstoff, Alkyl($C_1$-$C_4$), Halogenalkyl-($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)amino, Alkyl($C_1$-$C_4$)sulfonyl, OH, SH, $NH_2$ stehen, erhält man, wenn man

2,3-Diamino-maleinsäurenitril ("DAMN") der Formel

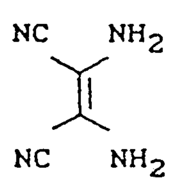

(II)

in molaren Mengen in einem geeigneten Verdünnungsmittel mit einem Aldehyd der Formel

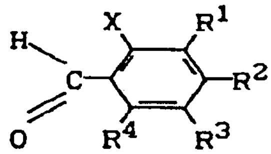

(III)

in welcher X, $R^1$, $R^2$, $R^3$ und $R^4$ die oben angegebene Bedeutung besitzen, umsetzt.

Überraschenderweise zeigen die teilweise bekannten Azomethine der Formel (I) eine Wirksamkeit gegen pflanzenschädigend Milben. Eine derartige Wirkung ist von der Stoffklasse der Azomethine des Diaminomaleinsäurenitrils bisher noch nicht bekannt geworden. Die Verwendung der Verbindungen der Formel (I) als Akarizide stellt somit eine Bereicherung des Standes der Technik dar.

Die Verbindungen der allgemeinen Formel (I) können in den stereoisomeren cis- und/oder trans-Formen vorliegen, z.B.

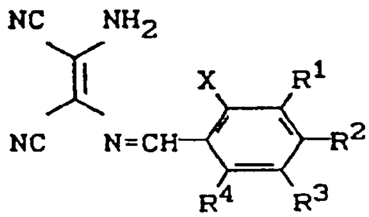

I'

bzw.

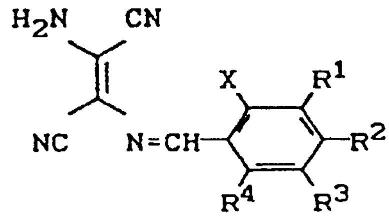

I''

Vorzugsweise liegen die Verbindungen jedoch in der cis-Form (I') vor.

Die Azomethine sind durch die Formel (I) allgemein definiert.

Besonders bevorzugt verwendet werden Verbindungen der allgemeinen Formel Ia

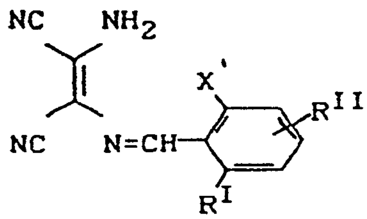

(Ia)

in der

X und $R^I$ gleich oder verschieden sein können und für Fluor, Chlor, Brom, Iod oder $CF_3$ stehen

und $R^{II}$ für

Wasserstoff, Chlor, Brom, Iod, Cyan, Nitro, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Pentafluorethyl, Trifluorethyl, Trifluormethylsulfon, Trifluormethoxy, Trichlormethoxy, Pentafluorethoxy, Dimethylamino, Diethylamino, Di-β-chlorethylamino, Di-β-hydroxyethylamino steht.

Ganz besonders bevorzugt verwendet werden Verbindungen der Formel Ib

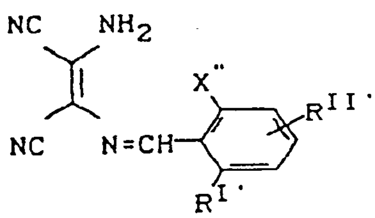

(Ib)

in der

X'' und $R^{I'}$ gleich oder verschieden sein können und für Chlor oder Brom stehen

und $R^{II'}$ für Chlor, Brom, Iod, Wasserstoff, Cyan, Nitro, Methyl, Ethyl, n- und i-Propyl, Methoxy, Ethoxy, Methoxycarbonyl, Ethoxycarbonyl, Trifluormethyl, Trichlormethyl, Dimethylamino, Diethylamino, Trifluormethylsulfon

steht.

Ganz besonders bevorzugt verwendet werden Verbindungen der Formel Ic

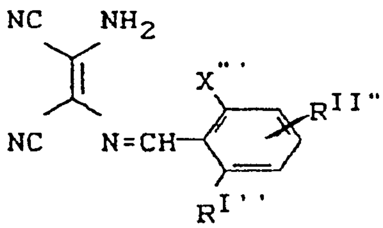

(Ic)

in der
$X'''$ und $R^{I''}$ gleich oder verschieden sein können und für Chlor oder Brom stehen,
und $R^{II''}$ für Wasserstoff, Chlor, Brom, Methyl, Cyano, Trichlormethyl, Trifluormethyl, Methoxy, Trifluormethoxy, Trichlormethoxy oder Trifluormethylsulfon steht.

Außerordentlich bevorzugt verwendet werden Verbindungen der allgemeinen Formel Id

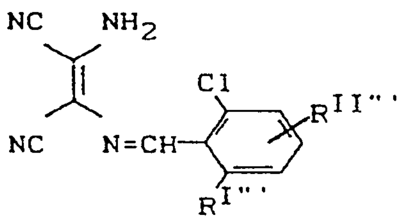

(Id)

in der
$R^{I'''}$ für Chlor oder Brom steht und
$R^{II'''}$ für Wasserstoff, Chlor, Methyl, Trichlormethyl, Methoxy, Trichlormethoxy, Trifluormethoxy steht.

Außergewöhnlich bevorzugt verwendet wird die Verbindung der Formel

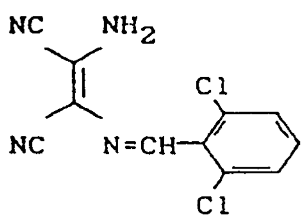

Im einzelnen seien außer den bei den Herstellungsbeispielen genannten Verbindungen die folgenden Azomethine der allgemeinen Formel (I) genannt:

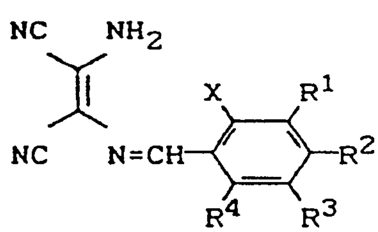

(I)

| X | $R^1$ | $R^2$ | $R^3$ | $R^4$ |
|---|---|---|---|---|
| Br | H | H | H | Br |
| Cl | $CH_3$ | H | H | Cl |
| Cl | $C_2H_5$ | H | H | $CH_3$ |
| Cl | $CH_3$ | H | H | $CH_3$ |
| Br | H | Br | H | H |
| Br | H | H | Br | H |
| Cl | H | H | H | Br |
| Cl | H | $C_2H_5$ | H | $CH_3$ |
| Cl | H | H | H | $C_2H_5$ |
| $CF_3$ | H | H | H | Cl |
| $CF_3$ | H | H | H | $CF_3$ |
| Cl | H | F | H | Cl |

Verwendet man beispielsweise 2,3-Diaminomaleinsäurenitril (DAMN) und 2,4-Dichlorbenzaldehyd als Ausgangsprodukte, so kann das Verfahren zur Herstellung von Verbindungen der Formel (I) wie folgt dargestellt werden :

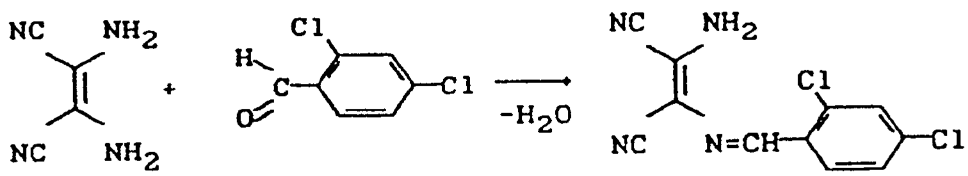


Die zur Durchführung des Verfahrens zur Herstellung der Verbindungen (I) als Ausgangsstoffe benötigten Aldehyde sind durch die Formeln (III) allgemein definiert. Es handelt sich im wesentlichen um bekannte Verbindungen. Siehe dazu z.B. O. Bayer, in Houben-Weyl, Bd. VII/1, S. 16-36 (1954).

Die weitere Ausgangsverbindung 2,3-Diaminomaleinsäurenitril (II) ist ebenfalls literaturbekannt und im Handel erhältlich.

Als Verdünnungsmittel zur Durchführung des Verfahrens zur Herstellung von Verbindungen (I) kommen vorzugsweise polare organische Lösungsmittel in Frage, wie z.B. Alkohole (insbesondere Methanol, Ethanol, Propanol (n und i)), Dimethylformamid (DMF), Dimethylsulfoxid (DMSO), Hexemethylphosphorsäuretriamid (HMPT), Acetonitril.

Die Reaktionstemperatur liegt im allgemeinen im Bereich zwischen ca. 0°C bis maximal zum Siedepunkt des jeweiligen Lösungsmittels, insbesondere wird die Reaktion bei Temperaturen von ca. 20 bis ca. 100°C durchgeführt.

Die Umsetzung wird vorzugsweise bei Normaldruck durchgeführt.

Zur Durchführung des Verfahrens zur Herstellung der Verbindungen (I) setzt man bevorzugt äquimolare Mengen der Reaktionspartner (II) und (III) miteinander um. Es kann aber auch ein leichter Überschuß eines der beiden Reaktionspartner angewendet werden.

In einer bevorzugten Ausführungsform gibt man bei Raumtemperatur die beiden Reaktionspartner im Verdünnungsmittel zusammen und erhitzt anschließend zum Rückfluß.

Die Aufarbeitung erfolgt nach üblichen Methoden, nach dem Abkühlen wird das Reaktionsprodukt vorzugsweise abgesaugt und nach an sich bekannten Methoden aufgearbeitet.

Die Wirkstoffe eignen sich bei guter Pflanzenverträglichkeit und günstiger Warmblütertoxizität zur Bekämpfung von pflanzenschädigenden Milben (Acarina), die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.,

Die Wirkstoffe (I) zeichnen sich durch eine hohe Wirksamkeit gegen pflanzenschädigende Milben, wie beispielsweise gegen die gemeine Spinnmilbe (Tetranychus urticae) aus.

Die Wirkstoffe können in die üblichen Formulierungen übergeführt werden, wie Lösungen, Emulsionen, Suspensionen, Pulver, Schäume, Pasten, Granulate, Aerosole,

Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier und/oder schaumerzeugende Mittel kommen in Frage : z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Wirkstoffe (I) können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden oder Fungiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die Wirkstoffe (I) können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäß verwendbaren Wirkstoffe eignen sich auch zur Bekämpfung von Milben, Zecken

usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Herstellungsbeispiele

Beispiel 1 (neue Verbindung)

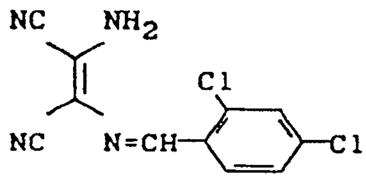

10,8 g 2,3-Diamino-maleinsäurenitril werden in 100 ml Ethanol mit 17,5 g 2,4-Dichlorbenzaldehyd zum Rückfluß erhitzt, Nach 3 Stunden läßt man die Lösung abkühlen und filtriert den Niederschlag ab.

Man erhält 25,7 g (= 97% d. Th.) der oben bezeichneten Verbindung.

Schmelzpunkt : 228°C (unter Zersetzung).

Beispiel 2

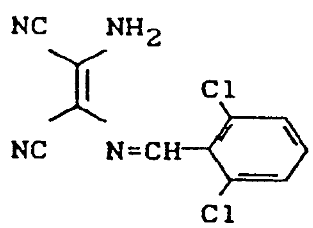

(Substanz ohne Angabe einer biologischen Wirksamkeit bekannt aus J. Org. Chem. 39, No. 16, 2344 (1974).

In 100 ml Acetonitril bringt man eine Mischung aus 10,8 g 2,3-Diaminomaleinsäurenitril und 17,5 g 2,6-Dichlorbenzaldehyd zum Sieden. Die Dünnschichtchromatographie zeigt nach 4 Stunden das Reaktionsende an. Nach dem Abkühlen saugt man 24,2 g (91% d. Th.) der Titelverbindung ab. Die Substanz (gelbgrüne Nadeln) schmilzt bei 191°C.

In Analogie zu den vorgenannten Beispielen wurden die folgenden neuen Verbindungen erhalten :

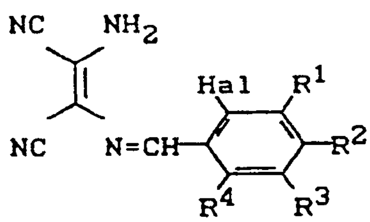

(I)

| Bsp. Nr. | Hal | $R^1$ | $R^2$ | $R^3$ | $R^4$ | Schmelzpunkt oder IR-Daten |
|---|---|---|---|---|---|---|
| 3 | Cl | H | $CF_3$ | H | H | 189 |
| 4 | Cl | H | H | H | H | 190 (Zers.) |
| 5 | Cl | H | Cl | H | Cl | 234 (Zers.) |
| 6 | Cl | Cl | $CF_3$ | H | Cl | 198 (Zers.) |
| 7 | Cl | H | $COOCH_3$ | H | Cl | 179 |
| 8 | Cl | $CF_3$ | Cl | H | H | 113 |
| 9 | Br | H | Br | H | Br | 304 |
| 10 | Cl | H | Br | H | Cl | 3400/3300 ($NH_2$) |
| 11 | Cl | H | Cl | H | Br | 2280/2230 (CN) |
| 12 | Cl | H | $CF_3$ | H | Cl | 2300/2240 (CN) |
| 13 | Cl | H | $OCF_3$ | H | Cl | 3430/3310 ($NH_2$) |
| 14 | Cl | H | $OCCl_3$ | H | Cl | 3420/3300 ($NH_2$) |
| 15 | Cl | H | $OCF_3$ | H | H | 2250/2210 (CN) |
| 16 | Cl | H | $OCCl_3$ | H | H | 1605 (C=C) |
| 17 | Cl | H | $OCH_3$ | H | Cl | 3430/3300 ($NH_2$) |
| 18 | Cl | H | $OCH_3$ | H | H | 2240/2200 (CN) |
| 19 | Cl | H | $SCF_3$ | H | Cl | 2250/2210 (CN) |
| 20 | Cl | H | $SO_2CF_3$ | H | Cl | 1320 ($-SO_2-$) |
| 21 | Cl | H | $SCF_3$ | H | H | 3400/3300 ($NH_2$) |
| 22 | Cl | H | $SO_2CF_3$ | H | H | 1315 ($-SO_2-$) |
| 23 | Cl | H | H | H | $CH_3$ | 1370 ($CH_3$) |
| 24 | Cl | H | $CF_3$ | H | $CH_3$ | 1375 ($CH_3$) |
| 25 | Cl | H | $OCF_3$ | H | $CH_3$ | 1380 ($CH_3$) |
| 26 | Cl | H | $SO_2CF_3$ | H | $CH_3$ | 1375 ($CH_3$) |
| 27 | Cl | H | $SCF_3$ | H | $CH_3$ | 1375 ($CH_3$) |
| 28 | Cl | H | H | H | CN | 2210/2250/2200 (CN) |
| 29 | Cl | H | CN | H | Cl | 2220/2240/2250 (CN) |
| 30 | Cl | H | H | $NO_2$ | H | 266 |
| 31 | F | F | F | F | F | 198 |
| 32 | Cl | H | H | H | F | 184 |
| 33 | $CF_3$ | H | H | H | $CF_3$ | 190 |
| 34 | F | H | H | H | F | 217 |
| 35 | F | $CF_3$ | F | F | F | 152 |
| 36 | Cl | H | Cl | $OCF_3$ | Cl | 163 |

Beispiel A

Tetranychus-Test (resistent)

Lösungsmittel : 7 Gewichtsteile Dimethylformamid
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in %. bestimmt. Dabei bedeutet 100%, daß alle Spinnmilben abgetötet wurden ; 0% bedeutet, daß keine Spinnmilben abgetötet wurden.

Bei diesem Test zeigen z.B. die folgenden Verbindungen der Herstellungsbeispiele eine hervorragende Wirkung gegen Tetranychus urticae (2)

## Tabelle

(pflanzenschädigende Milben)

Tetranychus Test

| Wirkstoffe | Wirkstoff-konz. in % | Abtötungsgrad nach 7 Tagen in % |
|---|---|---|
| 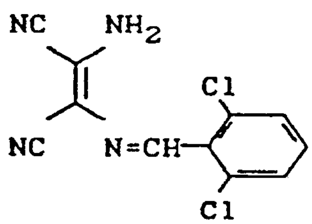 (2) | 0,1 | 98 |

## Ansprüche

1) Mittel gegen pflanzenschädigende Milben, gekennzeichnet durch einen Gehalt an mindestens einem Azomethin des 2,3-Diaminomaleinsäurenitrils der Formel

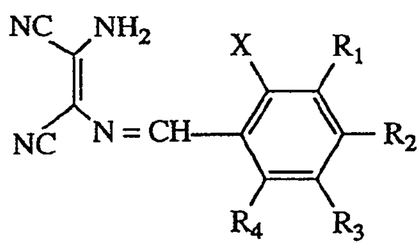

(I)

in welcher
X und $R^4$ gleich oder verschieden sein können und für Fluor, Chlor, Brom, Iod, $CF_3$ oder CN stehen,
$R^1$, $R^2$ und $R^3$ gleich oder verschieden sein können und für Wasserstoff, Alkyl($C_1$-$C_4$), Halogenakyl-($C_1$-$C_4$), Alkoxy($C_1$-$C_4$), Halogenalkoxy($C_1$-$C_4$), Halogen, CN, $NO_2$, Dialkyl($C_1$-$C_4$)amino, Alkoxy($C_1$-$C_4$)carbonyl, Alkyl($C_1$-$C_4$)thio, Alkyl($C_1$-$C_4$)thionyl, Dihalogenalkyl($C_1$-$C_4$)amino, Alkyl($C_1$-$C_4$)sulfonyl, OH, SH, $NH_2$ stehen, im Gemisch mit Streckmitteln unter Zusatz von oberflächenaktiven Mitteln

2) Mittel gegen pflanzenschädigende Milben gemäß Anspruch 1, dadurch gekennzeichnet, daß sie mindestens ein Azomethin des 2,3-Diaminomaleinsäurenitrils der Formel

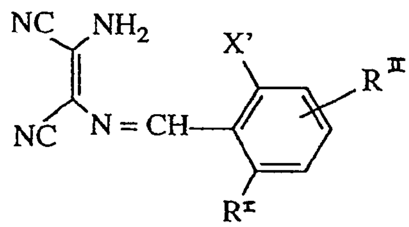

(I a)

in der
X'und $R^I$ gleich oder verschieden sein können und für Fluor, Chlor, Brom, Iod oder $CF_3$ stehen und
$R^{II}$ für Wasserstoff, Chlor, Brom, Iod, Cyan, Nitro, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Pentafluorethyl, Trifluorethyl, Trifluormethylsulfonyl, Trifluormethoxy, Trichlormethoxy, Pentafluorethoxy, Dimethylamino, Diethylamino, Di-β-chlorethylamino, Di-β-hydroxyethylamino steht, im Gemisch mit Streckmitteln unter Zusatz von oberflächenaktiven Mitteln enthalten

3) Mittel gegen pflanzenschädigende Milben gemäß Anspruch 1 oder 2, dadurch gekennzeichnet, daß sie das Azomethin des 2,3-Diaminomaleinsäurenitrils der Formel

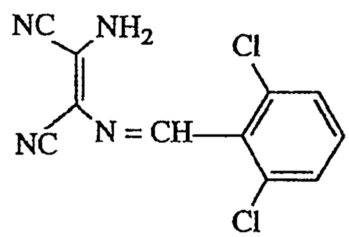

im Gemisch mit Streckmitteln unter Zusatz von oberflächenaktiven Mitteln enthalten

4) Verfahren zur Bekämpfung von pflanzenschädigenden Milben (Acarina), dadurch gekennzeichnet, daß man Azomethine des 2,3-Diaminomaleinsäurenitrils der Formel (I) auf planzenschädigende Milben und/oder ihren Lebensraum einwirken läßt.

5) Verwendung von Azomethinen des 2,3-Diaminomaleinsäurenitrils der Formel (I) zur Bekämpfung von pflanzenschädigenden Milben (Acarina).

6) Verfahren zur Herstellung von Mitteln gegen pflanzenschädigende Milben, dadurch gekennzeichnet, daß man Azomethine des 2,3-Diaminomaleinsäurenitrils der Formel (I) mit Streckmitteln und ober lächenaktiven Mitteln vermischt.

## Claims

1) Agents against mites which damage plants, characterized in that they contain at least one azomethine of 2,3-diaminomaleic acid nitrile of the formula

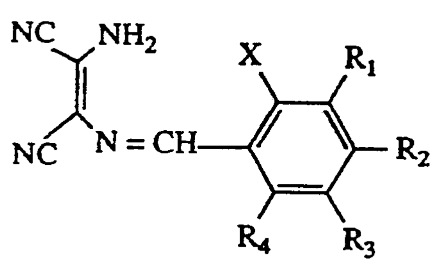

(I)

in which
X and $R^4$ can be identical or different and represent fluorine, chlorine, bromine, iodine, $CF_3$ or CN, and
$R^1$, $R^2$ and $R^3$ represent hydrogen, alkyl($C_1$-$C_4$), halogenoalkyl-($C_1$-$C_4$), alkoxy($C_1$-$C_4$), halogenoalkoxy($C_1$-$C_4$), halogen, CN, $NO_2$, dialkyl($C_1$-$C_4$)amino, alkoxy($C_1$-$C_4$) carbonyl, alkyl($C_1$-$C_4$)thio, alkyl($C_1$-$C_4$)thionyl, dihaloge-

no($C_1$-$C_4$)amino, alkyl($C_1$-$C_4$)sulphonyl, OH, SH, $NH_2$, in a mixture with extenders, with the addition of surface-active agents.

2) Agents against mites which damage plants, according to Claim 1, characterized in that they contain at least one azomethine of 2,3-diaminomaleic acid nitrile of the formula

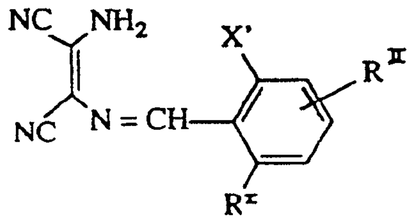

(I a)

in which
X′ and $R^1$ can be identical or different and represent fluorine, chlorine, bromine, iodine or $CF_3$ and
$R^{11}$ represents hydrogen, chlorine, bromine, iodine, cyano, nitro, methyl, ethyl, n- or i-propyl, n-, i-, s- or t-butyl, methoxy, ethoxy, methoxycarbonyl, ethoxycarbonyl, trifluoromethyl, trichloromethyl, pentafluoroethyl, trifluoroethyl, trifluoromethylsulphonyl, trifluoromethoxy, trichloromethoxy, pentafluoroethoxy, dimethylamino, diethylamino, di-β-chloroethylamino or di-β-hydroxyethlamino, in a mixture with extenders, with the addition of surface-active agents.

3) Agents against mites which damage plants according to Claim 1 or 2, characterized in that they contain the azomethine of 2,3-diaminomaleic acid nitrile of the formula

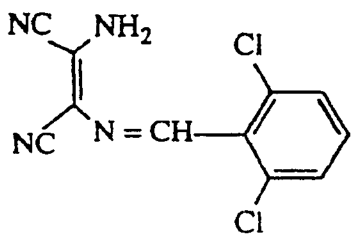

in a mixture with extenders with the addition of surface-active agents.

4) Method of combating mites (Acarina) which damage plants, characterized in that azomethines of 2, 3-diaminomaleic acid nitrile of the formula (I) are allowed to act on mites which damage plants and/or on their environment.

5) Use of azomethines of 2,3-diaminomaleic acid nitrile of the formula (I) for combating mites (Acarina) which damage plants.

6) Process for the preparation of agents against mites which damage plants, characterized in that azomethines of 2,3-diaminomaleic acid nitrile of the formula (I) are mixed with extenders and/or surface-active agents.

## Revendications

1) Compositions contre des acariens parasites des plantes, caractérisées par une teneur en au moins une azométhine du nitrile d'acide 2,3-diaminomaléique de formule

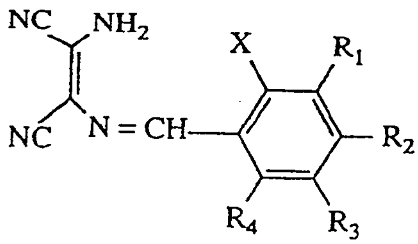

(I)

dans laquelle

X et $R^4$ peuvent être identiques ou différents et représentent le fluor, le chlore, le brome, l'iode, $CF_3$ ou CN,

$R^1$, $R^2$ et $R^3$ peuvent être identiques ou différents et représentent l'hydrogène, un groupe alkyle en $C_1$ à $C_4$, halogénalkyle en $C_1$ à $C_4$, alkoxy en $C_1$ à $C_4$, halogénalkoxy en $C_1$ à $C_4$, un halogène, un groupe CN, $NO_2$, di-(alkyle en $C_1$ à $C_4$)-amino, (alkoxy en $C_1$ à $C_4$)carbonyle, alkylthio en $C_1$ à $C_4$, alkylthionyle en $C_1$ à $C_4$, dihalogénalkylamino en $C_1$ à $C_4$, alkylsulfonyle en $C_1$ à $C_4$, OH, SH, $NH_2$, en mélange avec des diluants, avec addition d'agents tensio-actifs.

2) Compositions contre des acariens parasites des plantes suivant la revendication 1, caractérisées en ce qu'elles contiennent au moins une azométhine du nitrile d'acide 2,3-diaminomaléique de formule

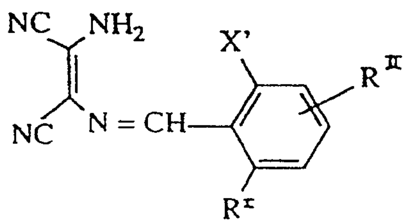

(I a)

dans laquelle

X' et $R^I$ peuvent être identiques ou différents et représentent le fluor, le chlore, le brome, l'iode ou un groupe $CF_3$ et

$R^{II}$ représente l'hydrogène, le chlore, le brome, l'iode, un groupe cyano, nitro, méthyle, éthyle, n-propyle, isopropyle, n-butyle, isobutyle, sec.-butyle, tertio-butyle, méthoxy, éthoxy, méthoxycarbonyle, éthoxycarbonyle, trifluorométhyle, trichlorométhyle, pentafluoréthyle, trifluoréthyle, trifluorométhylsulfonyle, trifluorométhoxy, trichlorométhoxy, pentafluoréthoxy, diméthylamino, diéthylamino, di-β-chloréthylamino, di-β-hydroxyéthylamino, en mélange avec des diluants, avec addition d'agents tensio-actifs.

3) Compositions contre des acariens parasites des plantes suivant la revendication 1 ou 2, caractérisées en ce qu'elles contiennent l'azométhine du nitrile d'acide 2,3-diaminomaléique de formule

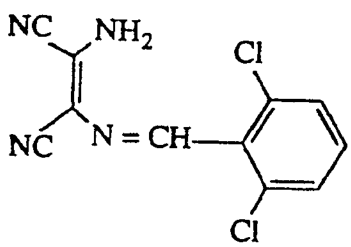

en mélange avec des diluants, avec addition d'agents tensio-actifs.

4) Procédé pour combattre des acariens (Acarina) parasites des plantes, caractérisé en ce qu'on fait agir des azométhines du nitrile d'acide 2,3-diaminomaléique de formule (I) sur des acariens parasitant les plantes et/ou sur leur milieu.

5) Utilisation d'azométhines du nitrile d'acide 2,3-diaminomaléique de formule (I) pour combattre des acariens (Acarina) parasitant les plantes.

6) Procédé de préparation de compositions contre des acariens parasitant les plantes, caractérisé en ce

qu'on mélange des azométhines du nitrile d'acide 2,3-diaminomaléique de formule (I) avec des diluants et des agents tensio-actifs.